(19) Europäisches Patentamt
European Patent Office
Office européen des brevets

(11) Veröffentlichungsnummer: **0 005 250**
**B1**

# (12) EUROPÄISCHE PATENTSCHRIFT

(45) Veröffentlichungstag der Patentschrift: **12.08.81**

(51) Int. Cl.³: **C 07 D 233/60**, A 61 K 31/415

(21) Anmeldenummer: **79101288.3**

(22) Anmeldetag: **30.04.79**

## (54) Hydroxypropyl-imidazole, Verfahren zu ihrer Herstellung sowie diese enthaltende Arzneimittel.

(30) Priorität: **10.05.78 DE 2820489**
**26.07.78 DE 2832677**

(43) Veröffentlichungstag der Anmeldung: **14.11.79 Patentblatt 79/23**

(45) Bekanntmachung des Hinweises auf die Patenterteilung: **12.08.81 Patentblatt 81/32**

(84) Benannte Vertragsstaaten: **BE CH DE FR GB IT NL SE**

(56) Entgegenhaltungen:
**DE-A-2 623 129**
**DE-A-2 732 750**
**DE-A-2 736 122**

(73) Patentinhaber: **BAYER AG, Zentralbereich Patente, Marken und Lizenzen, D-5090 Leverkusen 1, Bayerwerk (DE)**

(72) Erfinder: **Regel, Erik, Bergerheide 72a, D-5600 Wuppertal 1 (DE)**
Erfinder: **Büchel, Karl Heinz, Prof. Dr., Bergerheide 62, D-5600 Wuppertal 1 (DE)**
Erfinder: **Haller, Ingo, Dr., Viktoriastrasse 99, D-5600 WUppertal 1 (DE)**
Erfinder: **Plempel, Manfred, Dr., Pahlkestrasse 5, D-5600 Wuppertal 1 (DE)**

EP 0 005 250 B1

Anmerkung. Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Hydroxypropyl-imidazole, Verfahren zu ihrer Herstellung sowie diese enthaltende Arzneimittel

Die vorliegende Erfindung betrifft neue Hydroxypropylimidazole, mehrere Verfahren zu ihrer Herstellung sowie ihre Verwendung als Arzneimittel, insbesondere als Antimykotika.

Es ist bereits bekanntgeworden, daß 1-($\beta$-Aryl)-ethylimidazol-Derivate, wie insbesondere das 1-[2,4-Dichlor-$\beta$-(2,4-dichlorbenzyloxy)-phenethyl]-imidazolnitrat (MICONAZOL$^R$), gute antimykotische Wirkung aufweisen (vergleiche DE-AS 1 940 388). Jedoch ist deren Wirkung in-vivo, wie insbesondere gegen Candida, nicht immer befriedigend.

Auch aus DE-AS 2 623 129 sind antimykotische Substanzen bekanntgeworden. Jedoch reichen deren antimykotische Wirkungen, insbesondere gegenüber Candida albicans, Torulapsis glabrata, Trichophyton mentagr. und Microsporum camis, nicht aus.

Es wurde gefunden, daß die neuen Hydroxypropyl-imidazole der allgemeinen Formel

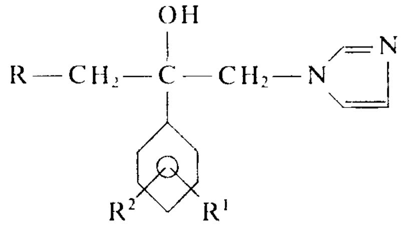

(I)

in welcher

R für gegebenenfalls durch Halogen oder Alkyl mit 1—4 C-Atomen substituiertes Phenyl, für Naphthyl oder für Tetrahydronaphthyl steht,

$R^1$ für gegebenenfalls durch Halogen oder Alkyl mit 1—4 C-Atomen substituiertes Phenyl oder für Cycloalkyl mit 5, 6 oder 7 C-Atomen steht und

$R^2$ für Wasserstoff steht, oder

$R^1$ und $R^2$ gemeinsam in o-Stellung zueinander für eine Tri-, Tetra- oder Pentamethylenbrücke stehen

und deren physiologisch verträglichen Säureadditionssalze starke antimykotische Eigenschaften aufweisen.

Weiterhin wurde gefunden, daß man die Hydroxypropylimidazole der Formel (I) enthält, wenn man

a) Imidazolyl-methyl-phenyl-ketone der Formel

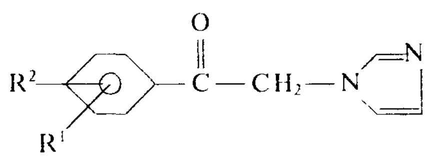

(II)

in welcher

$R^1$ und $R^2$ die oben angegebene Bedeutung haben,
mit einer Grignard-Verbindung der Formel

$$R-CH_2-Mg-X \quad (III)$$

in welcher

R die oben angegebene Bedeutung hat und
X für Halogen, insbesondere Chlor oder Brom steht,
in Gegenwart eines Verdünnungsmittels umsetzt, oder

b) 1-Halogen-propan-2-ole der Formel

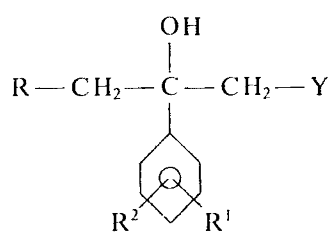

(IV)

in welcher

R, $R^1$ und $R^2$ die oben angegebene Bedeutung haben, und
Y für Halogen, insbesondere Chlor oder Brom stehen,

mit Imidazol, gegebenenfalls in Gegenwart eines Säurebindemittels und gegebenenfalls in Gegenwart eines Verdünnungsmittels, umsetzt. In manchen Fällen erweist es sich als vorteilhaft, anstelle des Imidazols ein Alkalisalz, wie das Natrium- oder Kaliumsalz, einzusetzen.

Die erfindungsgemäß erhältlichen Hydroxypropylimidazole können durch Umsetzen mit Säuren in die Salze überführt werden.

Überraschenderweise zeigen die erfindungsgemäßen Hydroxypropyl-imidazole neben einer guten antimykotischen in-vitro-Wirksamkeit eine bessere, therapeutisch nutzbare in-vivo-Wirksamkeit gegen Candida als das aus dem Stand der Technik bekannte 1-[2,4-Dichlor-β-(2,4-dichlorbenzyloxy)-phenäthyl]-imidazolnitrat, welches ein anerkannt gutes Mittel gleicher Wirkungsrichtung ist. Die erfindungsgemäßen Wirkstoffe stellen somit eine wertvolle Bereicherung der Pharmazie dar.

Von den erfindungsgemäßen Verbindungen der Formel (I) sind diejenigen bevorzugt, in denen R für Phenyl, das gegebenenfalls einfach oder zweifach durch Chlor, Fluor oder Methyl substituiert ist, für Naphthyl oder für Tetrahydronaphthyl steht; $R^1$ für Phenyl, das gegebenenfalls einfach oder zweifach durch Chlor, Brom, Fluor, Methyl, Äthyl, Isopropyl oder Isopropinyl substituiert ist, für Cyclopentyl oder für Cyclohexyl steht; und $R^2$ für Wasserstoff steht, oder $R^1$ und $R^2$ gemeinsam in ortho-Stellung zueinander für eine Tri-, Tetra- oder Pentamethylenbrücke stehen.

Verwendet man beispielsweise 4-Biphenylyl-(imidazol-1-yl-methyl)-keton und 4-Chlorbenzylmagnesiumchlorid als Ausgangsstoffe, so kann der Reaktionsablauf durch das folgende Formelschema wiedergegeben werden (Verfahren a):

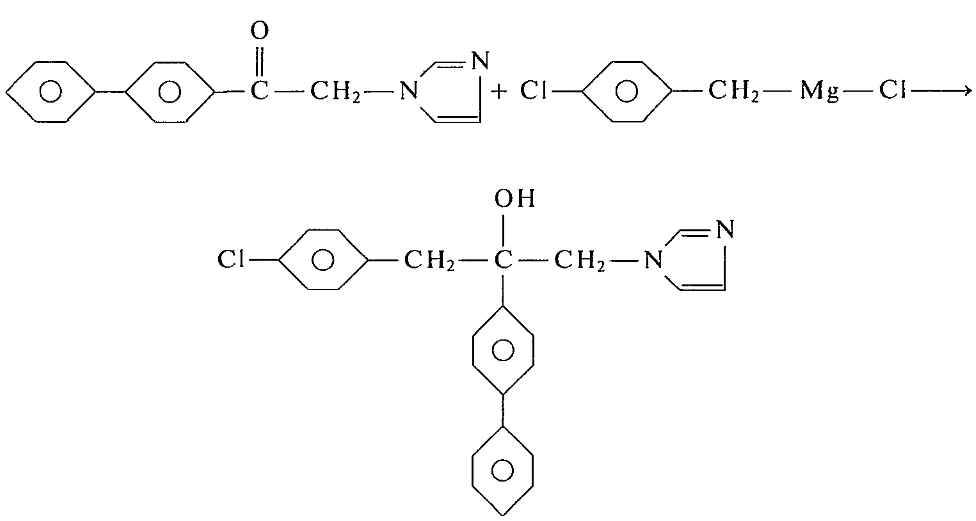

Verwendet man 2-(4-Biphenylyl)-3-chlor-1-(2,4-Dichlorphenyl)-propan-2-ol und Imidazol-natrium als Ausgangsstoffe, so kann der Reaktionsablauf durch das folgende Formelschema wiedergegeben werden (Verfahren b):

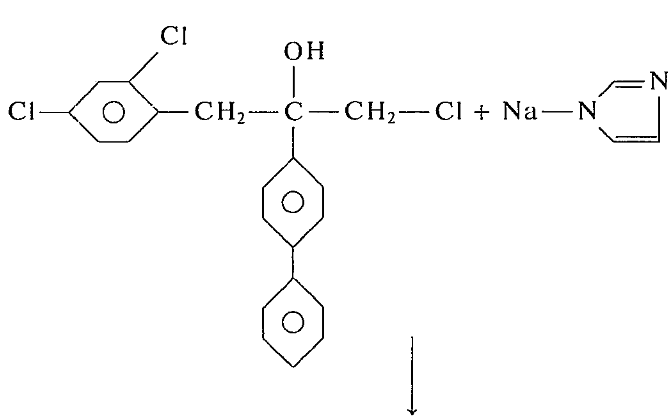

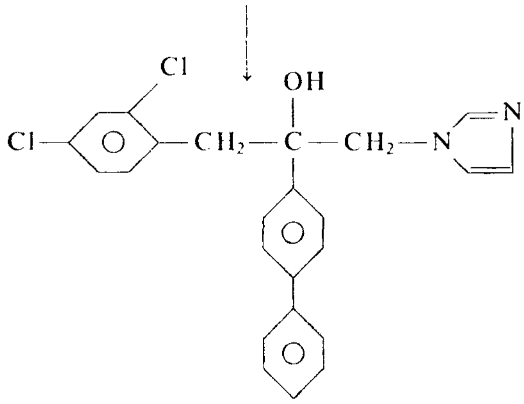

Die für die Verfahrensvariante (a) als Ausgangsstoffe zu verwendenden Imidazolylmethyl-phenyl-ketone sind durch die Formel (II) allgemein definiert. In dieser Formel stehen $R^1$ und $R^2$ vorzugsweise für die Reste, die bei den Verbindungen der Formel (I) bereits vorzugsweise genannt wurden.

Die Imidazolylmethyl-phenyl-ketone der Formel (II) sind noch nicht bekannt. Sie lassen sich jedoch in allgemein üblicher und bekannter Weise herstellen, indem man entsprechende Phenacylhalogenide der Formel

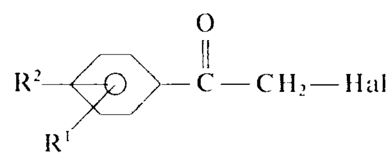

(VI)

in welcher

$R^1$ und $R^2$ die oben angegebene Bedeutung haben und
Hal für Chlor oder Brom steht,

mit Imidazol in Gegenwart eines Verdünnungsmittels, wie beispielsweise Dimethylformamid, und in Gegenwart eines säurebindenden Mittels, wie insbesondere einem Überschuß an Imidazol, bei Temperaturen zwischen 20 und 80°C umsetzt (vergleiche hierzu auch die Angaben in der US-Patentschrift 3 658 813 sowie die Herstellungsbeispiele).

Als Beispiele für die Ausgangsstoffe der Formel (II) seien genannt:

4-Biphenylyl-imidazol-1-yl-methyl-keton
4-(4'-Chlorphenylyl)-imidazol-1-yl-methyl-keton
2-Biphenylyl-imidazol-1-yl-methyl-keton
4-(2',4'-Dichlorbiphenylyl)-imidazol-1-yl-methyl-keton
4-Cyclohexylphenyl-imidazol-1-yl-methyl-keton
4-Cyclopentylphenyl-imidazol-1-yl-methyl-keton
4-Cycloheptylphenyl-imidazol-1-yl-methyl-keton
1,2,3,4-Tetrahydro-naphth-5-yl-imidazol-1-yl-methyl-keton
1,2,3,4-Tetrahydro-naphth-6-yl-imidazol-1-yl-methyl-keton
Indan-4-yl-imidazol-1-yl-methyl-keton

Die außerdem für die Verfahrensvariante (a) als Ausgangsstoffe zu verwendenden Grignard-Verbindungen sind durch die Formel (III) allgemein definiert. In dieser Formel steht R vorzugsweise für die Reste, die bei den Verbindungen der Formel (I) bereits vorzugsweise genannt wurden.

Die Grignard-Verbindungen der Formel (III) sind allgemein bekannte Verbindungen der organischen Chemie. Als Beispiele seien genannt:

Benzylmagnesiumchlorid, 4-Chlorbenzylmagnesiumchlorid,
2,4-Dichlorbenzylmagnesiumchlorid, 2,6-Dichlorbenzylmagnesiumchlorid,
2-Chlor-6-fluorbenzylmagnesiumchlorid, 2-Chlorbenzylmagnesiumchlorid,
3-Chlorbenzylmagnesiumchlorid, 3,4-Dichlorbenzylmagnesiumchlorid,
Naphth-2-yl-methylmagnesiumchlorid,
1,2,3,4-Tetrahydronaphth-6-yl-methylmagnesiumchlorid
sowie die entsprechende Bromide.

Die für die Verfahrensvariante (b) als Ausgangsstoffe zu verwendenden 1-Halogen-propan-2-ole sind durch die Formel (IV) allgemein definiert. In dieser Formel stehen R, $R^1$ und $R^2$ vorzugsweise für die Reste, die bei den Verbindungen der Formel (I) bereits vorzugsweise genannt wurden.

Die 1-Halogen-propan-2-ole der Formel (IV) sind noch nicht bekannt. Sie lassen sich jedoch in allgemein üblicher und bekannter Weise herstellen, indem man Ketone der Formel (VI) mit Grignard-Verbindungen der Formel (III) entsprechend der Verfahrensvariante (a) umsetzt (vergleiche hierzu auch die Angaben in der DE-OS 2 623 129 sowie die Herstellungsbeispiele).

Für die erfindungsgemäße Umsetzung nach Verfahren (a) kommen als Verdünnungsmittel alle für eine Grignard-Reaktion üblichen Lösungsmittel in Frage. Hierzu gehören vorzugsweise Äther, wie Diäthyläther oder Tetrahydrofuran sowie Gemische mit anderen organischen Solventien, wie z. B. Benzol.

Die Reaktionstemperaturen können beim Verfahren (a) in einem größeren Bereich variiert werden. Im allgemeinen arbeitet man zwischen etwa 20 bis etwa 120°C, vorzugsweise zwischen etwa 30 bis etwa 80°C.

Bei der Durchführung des Verfahrens (a) setzt man auf 1 Mol der Verbindung der Formel (II) vorzugsweise einen Überschuß von 3 bis 5 Mol der Grignard-Verbindung der Formel (III) ein. Die Isolierung der Verbindungen der Formel (I) erfolgt in üblicher und bekannter Weise.

Für die erfindungsgemäße Umsetzung nach Verfahren (b) kommen als Verdünnungsmittel vorzugsweise inerte organische Lösungsmittel in Frage. Hierzu gehören vorzugsweise Ketone, wie Diäthylketon, insbesondere Aceton und Methyläthylketon; Nitrile, wie Propionitril, insbesondere Acetonitril; Alkohole, wie Äthanol oder Isopropanol; Äther, wie Tetrahydrofuran oder Dioxan; aromatische Kohlenwasserstoffe, wie Benzol, Toluol und Dichlorbenzol; Formamide, wie insbesondere Dimethylformamid; und halogenierte Kohlenwasserstoffe, wie Methylenchlorid, Tetrachlorkohlenstoff oder Chloroform.

Wird das erfindungsgemäße Verfahren (b) in Gegenwart eines Säurebinders vorgenommen, so kann man alle üblicherweise verwendbaren anorganischen oder organischen Säurebinder zugeben, wie Alkalicarbonat, beispielsweise Natriumcarbonat, Kaliumcarbonat und Natriumhydrogencarbonat, oder wie niedere tertiäre Alkylamine, Cycloalkylamine oder Aralkylamine, beispielsweise Triäthylamin, N,N-Dimethylcyclohexylamin, Dicyclohexylmethylamin, N,N-Dimethylbenzylamin, weiterhin Pyridin und Diazabicyclooctan. Vorzugsweise verwendet man einen Überschuß an Imidazol.

Die Reaktionstemperaturen können beim Verfahren (b) in einem größeren Bereich variiert werden. Im allgemeinen arbeitet man zwischen etwa 30 bis etwa 200°C, vorzugsweise bei der Siedetemperatur des Lösungsmittels.

Bei der Durchführung des erfindungsgemäßen Verfahrens (b) setzt man auf 1 Mol der Verbindungen der Formel (IV) vorzugsweise 1 bis 2,5 Mol Imidazol und 1 bis 2,5 Mol Säurebinder ein. Bei Verwendung eines Alkalisalzes setzt man vorzugsweise auf 1 Mol der Verbindung der Formel (IV) 1 bis 1,5 Mol Alkalisalz ein. Zur Isolierung der Verbindungen der Formel (I) wird das Lösungsmittel abdestilliert, der Rückstand direkt oder nach Aufnahme mit einem organischen Solvens mit Wasser gewaschen, die organische Phase gegebenenfalls über Natriumsulfat getrocknet und im Vakuum vom Lösungsmittel befreit. Der Rückstand wird gegebenenfalls durch Destillation, Umkristallisation oder chromatographisch gereinigt.

Zur Herstellung von Säureadditionssalzen der Verbindungen der Formel (I) kommen alle physiologisch verträglichen Säuren in Frage. Hierzu gehören vorzugsweise die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure sowie Sulfonsäuren, wie z. B. p-Toluolsulfonsäure und 1,5-Naphthalindisulfonsäure.

Die Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Als Beispiele für besonders wirksame Vertreter der erfindungsgemäßen Wirkstoffe seien außer den Herstellungsbeispielen und den Beispielen der Tabelle 1 genannt:

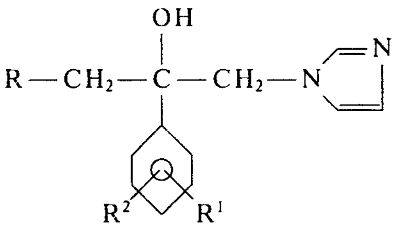

| R | $R^1$ | $R^2$ |
|---|---|---|
| 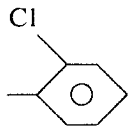 | 4 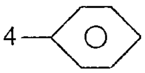 | H |
| 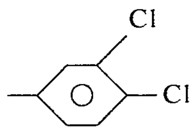 | 4 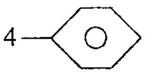 | H |
| 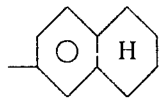 | 4 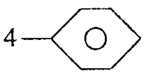 | H |
| 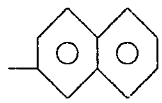 | 4 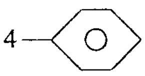 | H |
| 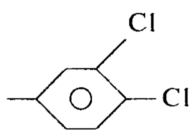 | 4 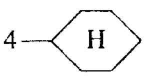 | H |
| 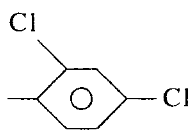 | 4 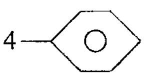 | H |
| 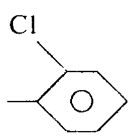 | 3,4 $-(CH_2)_4-$ | |
| 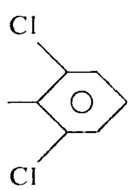 | 3,4 $-(CH_2)_3-$ | |
| 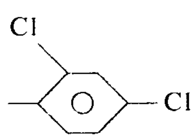 | 4 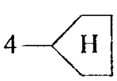 | H |
| 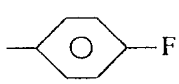 | 4 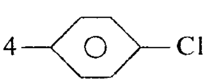 | H |

Fortsetzung

| R | $R^1$ | $R^2$ |
|---|---|---|
| 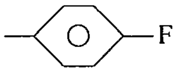 | 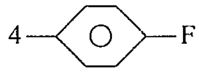 | H |
| 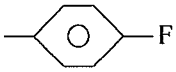 | 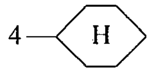 | H |
| 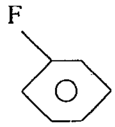 | 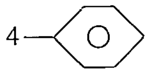 | H |
| 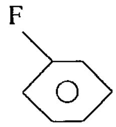 | 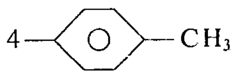 | H |
| 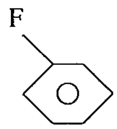 | 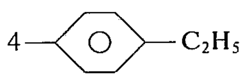 | H |
| 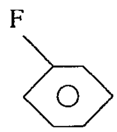 | 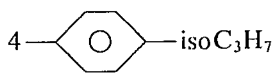 | H |
| 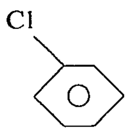 | 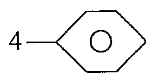 | H |
| 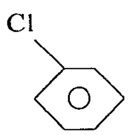 | 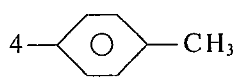 | H |
| 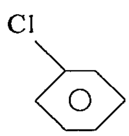 | 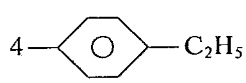 | H |
| 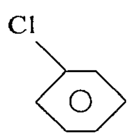 | 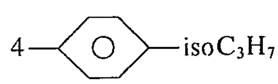 | H |

Die erfindungsgemäßen Verbindungen der Formel (I) und ihre Säureadditions-Salze weisen antimikrobielle, insbesondere antimykotische Wirkungen auf. Sie besitzen ein sehr breites antimykotisches Wirkungsspektrum, insbesondere gegen Dermatophyten und Sproßpilze sowie biphasische Pilze, z. B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indiaktionsgebiete in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder $^1/_2$, $^1/_3$ oder $^1/_4$ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quartenäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyäthylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyäthylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Äthylalkohol, Isopropylalkohol, Äthylcarbonat, Äthylacetat, Benzylalkohol, Benzylenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Äthylalkohol, Propylenglykol, Suspendiermittel, z. B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z. B. Pfefferminzöl und Eukalyptusöl und Süßmittel, z. B. Saccharin enthalten.

Die therapeutisch wirksamen Verbindungen sollen in den oben aufgeführten pharmazeutischen

Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Beispiel A

### Antimykotische in-vitro-Wirksamkeit

Versuchsbeschreibung:

Die in-vitro-Prüfungen wurden im Reihenverdünnungstest mit Keiminokula von durchschnittlich $5 \times 10^4$ Keimen/ml Substrat durchgeführt. Als Nährmedium dienten

a) für Dermatophyten und Schimmelpilze:
Sabouraud's milieu d'épreuve
b) für Hefen:
Fleischextrakt-Traubenzucker-Bouillon.

Die Bebrütungstemperatur betrug 20° C, die Bebrütungsdauer lag bei 24 bis 96 Stunden.

Bei diesen Versuchen zeigten die erfindungsgemäßen Verbindungen gute minimale Hemmkonzentrationen gegen die obengenannten Pilze.

## Beispiel B

### Antimikrobielle in-vivo-Wirksamkeit (oral) bei Mäuse-Candidose

Versuchsbeschreibung:

Mäuse vom Typ SPF-$CF_1$ wurden intravenös mit $1-2 \times 10^6$ logarithmisch wachsenden Candida-Zellen, die in physiologischer Kochsalzlösung suspendiert waren, infiziert. Eine Stunde vor und sieben Stunden nach der Infektion wurden die Tiere mit jeweils 50—100 mg/kg Körpergewicht der Präparate oral behandelt.

Ergebnis:

Unbehandelte Tiere starben 3 bis 6 Tage post infektionem. Die Überlebensrate am 6. Tag post infektionem betrug bei unbehandelten Kontrolltieren etwa 5%.

Das bekannte 1-[2,4-Dichlor-$\beta$-(2,4-dichlorbenzyloxy)-phenyläthyl]-imidazolnitrat (MICONAZOL®) zeigte bei dieser Dosierung keine Wirkung.

Dagegen zeigten die erfindungsgemäßen Verbindungen eine gute bis sehr gute Wirkung.

## Herstellungsbeispiele

### Beispiel 1

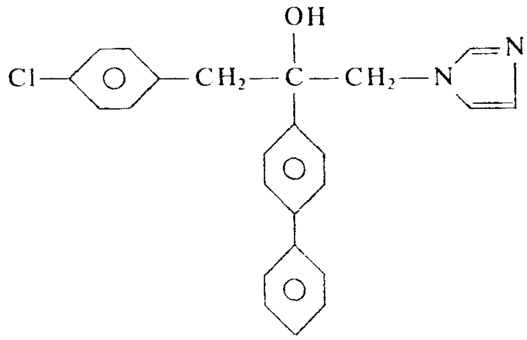

(Verfahren a)

Eine Lösung von 13,1 g (0,05 Mol) 4-Biphenylyl-(imidazol-1-yl-methyl)-keton in 500 ml Benzol wird zu einer 4-Chlorbenzylmagnesiumchlorid-Lösung, erhalten aus 6,1 g (0,25 Mol) Magnesium und 40,2 g (0,25 Mol) 4-Chlorbenzylchlorid in 150 ml Äther, getropft. Danach wird der Äther abdestilliert und das Reaktionsgemisch ca. 8 Stunden auf 80°C erhitzt. Die erkaltete Lösung wird auf eine Ammoniumchlorid-Lösung gegossen, die organische Phase abgetrennt, mit Wasser gewaschen und über Natriumsulfat getrocknet. Nach Abdestillieren des Benzols wird der verbleibende Kristallbrei mit Äther verrührt, die Kristalle werden abgesaugt und aus Acetonitril umkristallisiert. Man erhält 3,5 g (18% der Theorie) 2-(4-Biphenylyl)-1-(4-chlorphenyl)-3-(imidazol-1-yl)-propan-2-ol vom Schmelzpunkt 182°C.

### Herstellung des Ausgangsproduktes

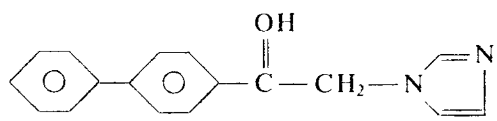

In eine Lösung von 48,2 g (0,7 Mol) Imidazol in 140 ml Dimethylformamid werden unter Kühlung 39 g (0,14 Mol) 4-Phenylphenacylbromid portionsweise eingetragen. Man läßt 15 Stunden nachreagieren und gießt die Lösung in Wasser. Die ausgeschiedene Kristallmasse wird aus Äthanol umkristallisiert. Man erhält 24 g (65% der Theorie) 4-Biphenylyl-(imidazol-1-yl-methyl)-keton vom Schmelzpunkt 198°C.

### Beispiel 2

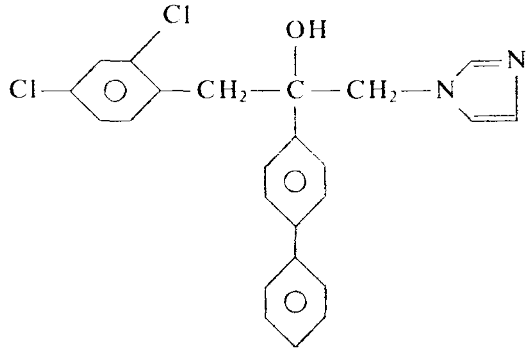

(Verfahren b)

Zu einer Suspension von 3,6 g (0,12 Mol) Natriumhydrid und 8,2 g (0,12 Mol) Imidazol in 200 ml Acetonitril wird eine Lösung von 39,15 g (0,1 Mol) 2-(4-Biphenylyl)-3-chlor-1-(2,4-dichlorphenyl)-propan-2-ol in 300 ml Acetonitril zugetropft. Nach 3stündigem Erhitzen auf 80°C wird das erkaltete Reaktionsgemisch filtriert, die Kristallmasse mit Wasser und mit Acetonitril gewaschen. Man erhält 32,5 g (77% der Theorie) 2-(4-Biphenylyl)-1-(2,4-dichlorphenyl)-3-(imidazol-1-yl)-propan-2-ol vom Schmelzpunkt 168° C.

Herstellung des Ausgangsproduktes

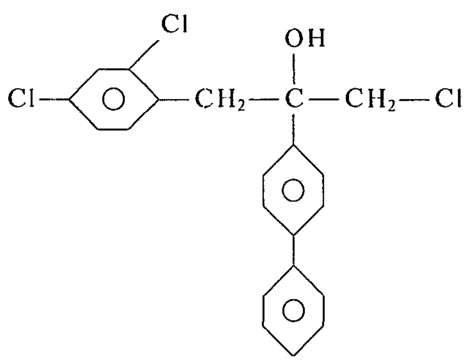

Zu einer Lösung von 0,6 Mol 2,4-Dichlorbenzylmagnesiumchlorid, erhalten aus 15,9 g (0,65 Mol) Magnesium und 117,3 g (0,6 Mol) 2,4-Dichlorbenzylchlorid in 300 ml Äther, werden 69,3 g (0,3 Mol) 4-Phenylphenacylchlorid portionsweise zugegeben. Anschließend wird das Reaktionsgemisch auf wäßrige Ammoniumchlorid-Lösung gegossen, die Ätherphase wird abgetrennt, mit Wasser gewaschen, über Natriumsulfat getrocknet und eingedampft. Das verbleibende Öl wird mit Petroläther extrahiert und die Petrolätherlösung eingedampft. Die Kristalle werden abfiltriert und getrocknet. Man erhält 62 g (53% der Theorie) 2-(4-Biphenylyl)-3-chlor-1-(2,4-dichlorphenyl)-propan-2-ol vom Schmelzpunkt 84° C.

Beispiel 3

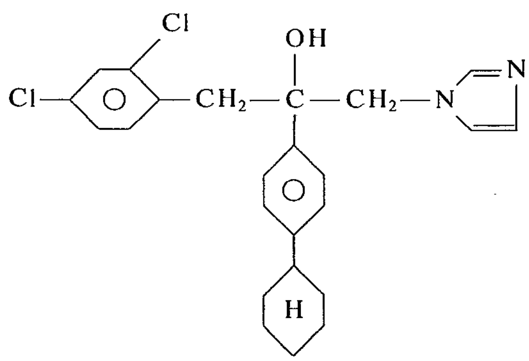

(Verfahren b)

Zu einer Suspension von 3,6 g (0,12 Mol) Natriumhydrid und 8,2 g (0,12 Mol) Imidazol in 200 ml Acetonitril wird eine Lösung von 39,8 g (0,1 Mol) 2-(4-Cyclohexylphenyl)-3-chlor-1-(2,4-dichlorphenyl)-propan-2-ol in 200 ml Acetonitril zugetropft. Nach dreistündigem Erhitzen auf 80° C wird filtriert, die Kristallmasse mit Wasser gewaschen und aus Acetonitril umkristallisiert. Man erhält 24,9 g (58% der Theorie) 2-(4-Cyclohexylphenyl)-1-(2,4-dichlorphenyl)-3-(imidazol-1-yl)-propan-2-ol vom Schmelzpunkt 142° C.

Herstellung des Ausgangsproduktes

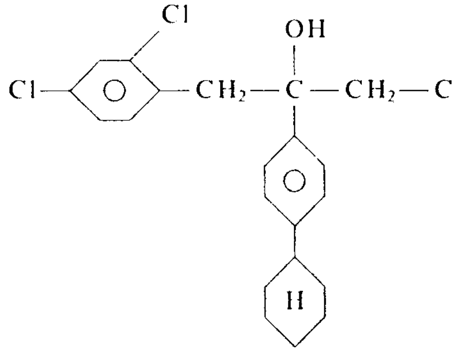

Zu einer Lösung von 0,4 Mol 2,4-Dichlorbenzylmagnesiumchlorid, erhalten aus 10,6 g (0,44 Mol) Magnesium und 78 g (0,4 Mol) 2,4-Dichlorbenzylchlorid in 150 ml Äther, wird eine Lösung von 47,3 g (0,2 Mol) 4-Cyclohexylphenacylchlorid in 200 ml Äther getropft. Das Reaktionsgemisch wird auf wäßrige Ammoniumchlorid-Lösung gegossen, die Äther-Phase wird abgetrennt, mit Wasser gewaschen, über Natriumsulfat getrocknet und eingedampft. Man erhält 79,5 g (99% der Theorie) 2-(4-Cyclohexyl)-3-chlor-1-(2,4-dichlorphenyl)-propan-2-ol vom Brechungsindex n = 1,5780.

In entsprechender Weise werden sowohl nach Verfahren a) als auch nach Verfahren b) die Verbindungen der nachfolgenden Tabelle 1 erhalten:

Tabelle 1

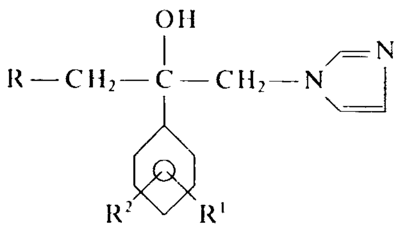

| Bsp. Nr. | R | $R^1$ | $R^2$ | Schmelzpunkt (°C) |
|---|---|---|---|---|
| 4 | 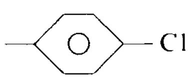 | 4 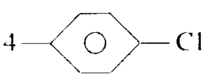 | H | 142 |
| 5 | 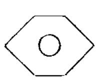 | 4 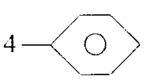 | H | 219 |
| 6 | 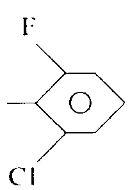 | 4 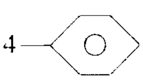 | H | 180 |
| 7 | 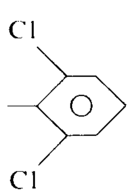 | 4 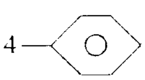 | H | 197 |
| 8 | 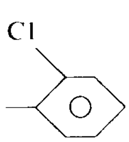 | 4 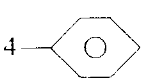 | H | 190 |

Fortsetzung

| Bsp. Nr. | R | $R^1$ | $R^2$ | Schmelzpunkt (°C) |
|---|---|---|---|---|
| 9 | 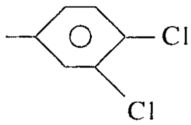 | 4 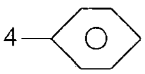 | H | 134 |
| 10 | 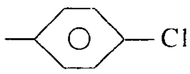 | 3,4—$(CH_2)_3$— | H | 135 |
| 11 | 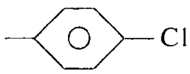 | 4 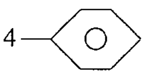 | H | 186 |
| 12 | 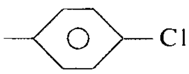 | 3,4—$(CH_2)_4$— | | 130 |
| 13 | 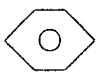 | 4 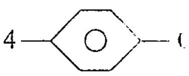 | H | 230 |
| 14 | 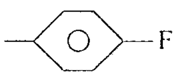 | 4 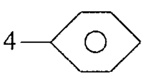 | H | 186 |
| 15 | 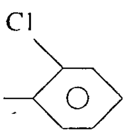 | 4 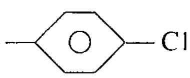 | H | 198 |
| 16 | 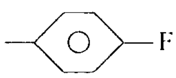 | 4 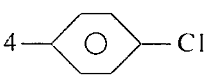 | H | 196 |
| 17 | 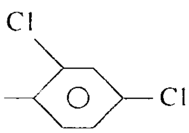 | 4 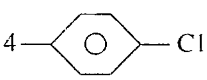 | H | 186 |
| 18 | 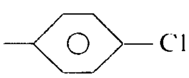 | 4 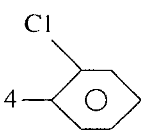 | H | 146 |
| 19 | 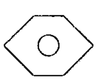 | 4 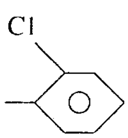 | H | 167 |
| 20 | 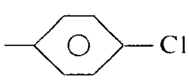 | 4 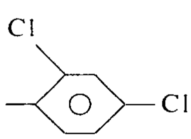 | H | 206 |
| 21 | 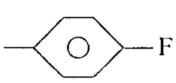 | 4 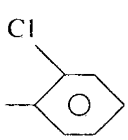 | H | 152 |

Fortsetzung

| Bsp. Nr. | R | $R^1$ | $R^2$ | Schmelzpunkt (°C) |
|---|---|---|---|---|
| 22 | 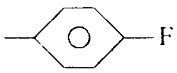 | 4 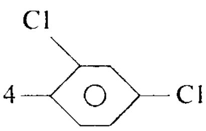 | H | 200 |
| 23 | 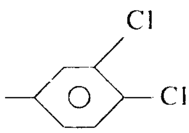 | 4 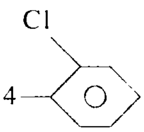 | H | 132 |
| 24 | 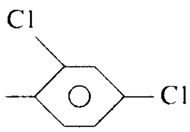 | 4 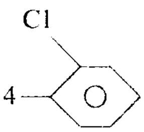 | H | 130 |
| 25 | 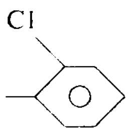 | 4 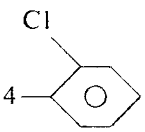 | H | 110 |
| 26 | 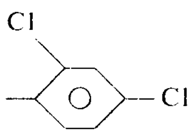 | 4 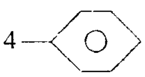 | H | 225 (× HCl) |
| 27 | 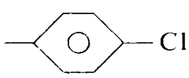 | 4 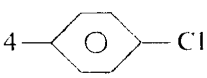 | H | 180 (× HCl) |
| 28 | 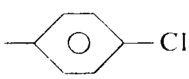 | 4 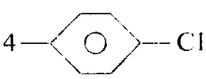 | H | 215 (× $H_2SO_4$) |
| 29 | 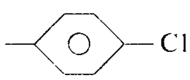 | 4 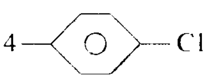 | H | 155 (× $HNO_3$) |
| 30 | 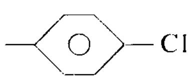 | 4 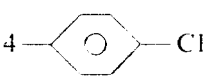 | H | 136 (× $CH_3COOH$) |
| 31 | 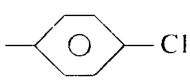 | 4 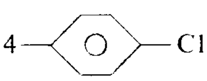 | H | 260 (× $H_3PO_4$) |

**Patentansprüche**

1. Hydroxypropyl-imidazole der allgemeinen Formel (I)

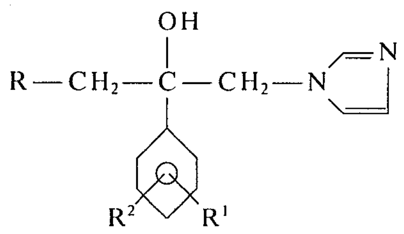

(I)

in welcher

R für gegebenenfalls durch Halogen oder Alkyl mit 1—4 C-Atomen substituiertes Phenyl, für Naphthyl oder für Tetrahydronaphthyl steht;
$R^1$ für gegebenenfalls durch Halogen oder Alkyl mit 1—4 C-Atomen substituiertes Phenyl oder für Cycloalkyl mit 5, 6 oder 7 C-Atomen steht; und
$R^2$ für Wasserstoff steht, oder
$R^1$ und $R^2$ gemeinsam in o-Stellung zueinander für eine Tri-, Tetra- oder Pentamethylenbrücke stehen,

und deren physiologisch verträglichen Säureadditionssalze.

2. Verfahren zur Herstellung von Hydroxypropyl-imidazolen der allgemeinen Formel (I) in Anspruch 1, dadurch gekennzeichnet, daß man

a) Imidazolyl-methyl-phenyl-ketone der Formel

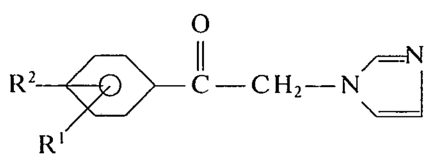

(II)

in welcher

$R^1$ und $R^2$ die in Anspruch 1 angegebene Bedeutung haben,
mit einer Grignard-Verbindung der Formel

$$R—CH_2—Mg—X \quad \text{(III)}$$

in welcher

R die in Anspruch 1 angegebene Bedeutung hat und
X für Halogen, insbesondere Chlor oder Brom steht,
in Gegenwart eines Verdünnungsmittels umsetzt, oder

b) 1-Halogen-propan-2-ole der Formel

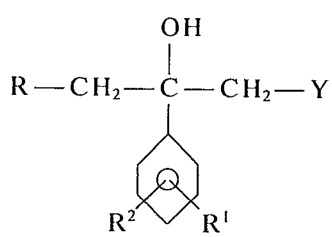

(VI)

in welcher

R, $R^1$ und $R^2$ die in Anspruch 1 angegebene Bedeutung haben, und
Y für Halogen, insbesondere Chlor oder Brom stehen,

mit Imidazol, gegebenenfalls in Gegenwart eines Säurebindemittels und gegebenenfalls in Gegenwart

eines Verdünnungsmittels, umsetzt und die dabei erhaltenen Verbindungen gegebenenfalls in ihre Salze überführt.

3. Arzneimittel, gekennzeichnet durch einen Gehalt an mindestens einem Hydroxypropyl-imidazol der allgemeinen Formel (I) gemäß Anspruch 1.

**Claims**

1. Hydroxypropyl-imidazoles of the general formula (I)

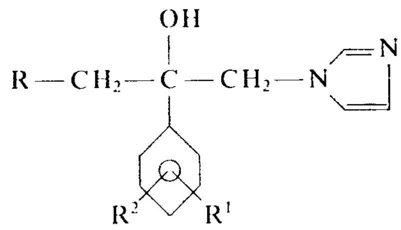

(I)

in which

R denotes phenyl optionally substituted by halogen or alkyl with 1–4 C atoms, naphthyl or tetrahydronaphthyl;
$R^1$ denotes phenyl optionally substituted by halogen or alkyl with 1–4 C atoms or cycloalkyl with 5, 6 or 7 C atoms; and
$R^2$ denotes hydrogen, or
$R^1$ and $R^2$ together, in the o-position relative to one another, denote a trimethylene, tetramethylene or pentamethylene bridge,

and physiologically acceptable acid addition salts thereof.

2. Process for the preparation of hydroxypropyl-imidazoles of the general formula (I) in claim 1, characterised in that

a) imidazolyl-methyl-phenyl ketones of the formula

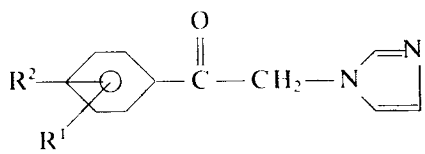

(II)

in which

$R^1$ and $R^2$ have the meaning indicated in claim 1,
are reacted with a Grignard compound of the formula

$$R-CH_2-Mg-X \quad (III)$$

in which

R has the meaning indicated in claim 1 and
X denotes halogen, in particular chlorine or bromine, in the presence of a diluent, or

b) 1-halogeno-propan-2-ols of the formula

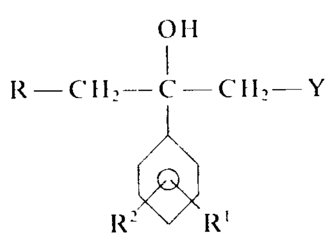

(IV)

in which

R, $R^1$ and $R^2$ have the meaning indicated in claim 1, and
Y denotes halogen, in particular chlorine or bromine,

are reacted with imidazole, optionally in the presence of an acid-binding agent and optionally in the presence of a diluent and the compounds thus obtained are optionally converted into their salts.

3. A medicament characterised in that it contains at least one hydroxypropyl-imidazole of the general formula (I) according to claim 1.

**Revendications**

1. Des hydroxypropyl-imidazoles de formule générale (I):

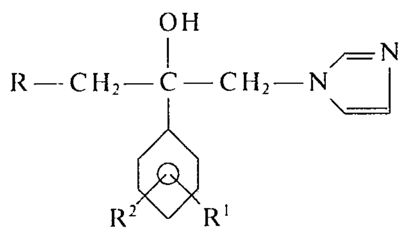

(I)

dans laquelle:

R est un groupe phényle éventuellement substitué par un halogène ou par un radical alkyle ayant 1 à 4 atomes de carbone, un groupe naphtyle ou un groupe tétrahydronaphtyle;
$R^1$ est un groupe phényle éventuellement substitué par un halogène ou par un radical alkyle ayant 1 à 4 atomes de carbone ou un groupe cycloalkyle ayant 5, 6 ou 7 atomes de carbone; et
$R^2$ est de l'hydrogène, ou
$R^1$ et $R^2$ forment ensemble, en position ortho l'un par rapport à l'autre, un pont triméthylénique, tétraméthylénique ou pentaméthylénique,

et leurs sels d'addition d'acides acceptables du point de vue physiologique.

2. Procédé de production d'hydroxypropyl-imidazoles de formule générale (I) suivant la revendication 1, caractérisé en ce qu'on fait réagir:

a) des imidazolyl-méthyl-phényl-cétones de formule:

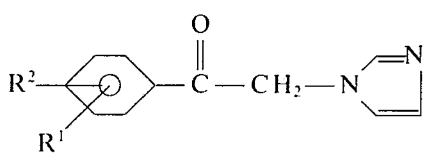

(II)

dans laquelle:

$R^1$ et $R^2$ ont la définition indiquée dans la revendication 1, avec un composé de Grignard de formule:

$$R-CH_2-Mg-X \quad (III)$$

dans laquelle:

R a la définition indiquée dans la revendication 1 et
X est un halogène, notamment le chlore ou le brome, en présence d'un diluant, ou

b) des 1-halogéno-propane-2-ols de formule:

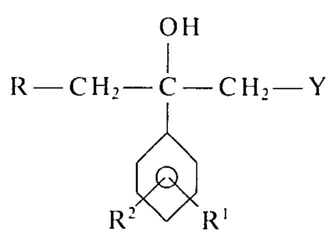

(IV)

dans laquelle:

R, $R^1$ et $R^2$ ont la définition indiquée dans la revendication 1 et
Y est un halogène, notamment le chlore ou le brome,

avec l'imidazole, éventuellement en présence d'un accepteur d'acide et en la présence éventuelle d'un diluant et les composés ainsi obtenus sont alors éventuellement transformés en leurs sels.

3. Médicament, caractérisé par une teneur en au moins un hydroxypropyl-imidazole de formule générale (I) suivant la revendication 1.